# FASCICULE DE BREVET EUROPEEN

(11) **EP 1 741 401 B1**
(45) Date de publication et mention de la délivrance du brevet: **09.09.2009**
(21) Numéro de dépôt: 06291091.4
(22) Date de dépôt: 03.07.2006
(51) Int. Cl.: A61B 19/00, G06K 7/00

(54) **Procédé de tri et de rangement d'instruments tels que des instruments chirurgicaux, et installation pour la mise en oeuvre de ce procédé**
Verfahren zur Sortierung und zum Aufbewahren von Instrumenten wie von chirurgischen Instrumenten und Anlage zur Durchführung dieses Verfahrens
Method for sorting and storing devices such as surgical devices and installation for implementing same

(30) Priorité: 04.07.2005 FR 0507081
(43) Date de publication de la demande: 10.01.2007
(73) Titulaire: De Uthemann, Cyril, 1253 Vandoeuvres (CH)
(72) Inventeur: Despres, Jean-Albert, 41300 Souesmes (FR)
(74) Mandataire: Delaveau, Sophie

(56) Documents cités:
- WO-A-03/105216
- DE-A1- 19 510 392
- US-A- 5 610 811
- US-A1- 2005 038 556

## Description

L'invention concerne un procédé de tri et de rangement d'instruments tels que des instruments chirurgicaux, devant être utilisés pour une opération prédéterminés, telle qu'une opération chirurgicale, et une installation pour la mise en oeuvre de ce procédé.

Un procédé de ce type, et une installation pour la mise en oeuvre de celui-ci, sont déjà connus par le brevet US 2005/0038556. Selon ce procédé, des instruments médicaux qui ont été soumis à une première étape de nettoyage sont envoyés à un poste d'identification pour pouvoir ensuite être transportés à un endroit de nettoyage définitif dépendant du type de l'instrument. L'identification des instruments se fait à l'aide d'une caméra CCD par lecture d'un code écrit sur la face extérieure des instruments.

Ce procédé présente l'inconvénient majeur que l'identification des instruments est insuffisamment fiable pour pouvoir être utilisé dans le cadre de la préparation des instruments nécessaires pour une opération chirurgicale où toute erreur pourrait être fatale. En effet, il suffit que la surface de l'instrument soit quelque peu souillée, pour que le code ne soit plus lisible, d'une façon absolument sûre.

L'invention a pour but de pallier cet inconvénient.

Pour attendre ce but, le procédé et l'installation selon l'invention utilise les caractéristiques qui sont indiquées dans les revendications respectivement 1 et 5, selon lesquelles le code d'identification d'un instrument est prévu sur un insert disposé à l'intérieur de l'instrument et qui est réalisé en un matériau plus opaque aux rayons X que le matériau constitutif de l'instrument et on effectue l'identification de l'instrument à l'aide de rayons X.

D'autres caractéristiques sont décrites dans des revendications dépendantes.

L'invention sera mieux comprise, et d'autres buts, caractéristiques, détails et avantages de celle-ci apparaîtront plus clairement au cours de la description explicative qui va suivre faite en référence aux dessins schématiques annexés donnés uniquement à titre d'exemple illustrant un mode de réalisation de l'invention et dans lesquels :
- la figure 1 est une vue schématique, en perspective, d'une installation selon l'invention ;
- la figure 2 est une vue de dessus d'un plateau de support d'un instrument selon l'invention ;
- la figure 3 est une vue éclatée, d'un plateau de support, selon la figure 2 ; et
- les figures 4 et 5 illustrent une configuration avantageuse des plateaux selon l'invention et d'un étage de stockage.

L'invention sera décrite ci-après dans son application à un procédé de tri d'instruments chirurgicaux destinés à des opérations chirurgicales. Bien entendu, la description est uniquement donnée à titre d'exemple, mais, de façon générale, l'invention est utilisable à toute opération qui implique une sélection et un rangement d'instruments spécifique pour la mise en oeuvre de l'opération.

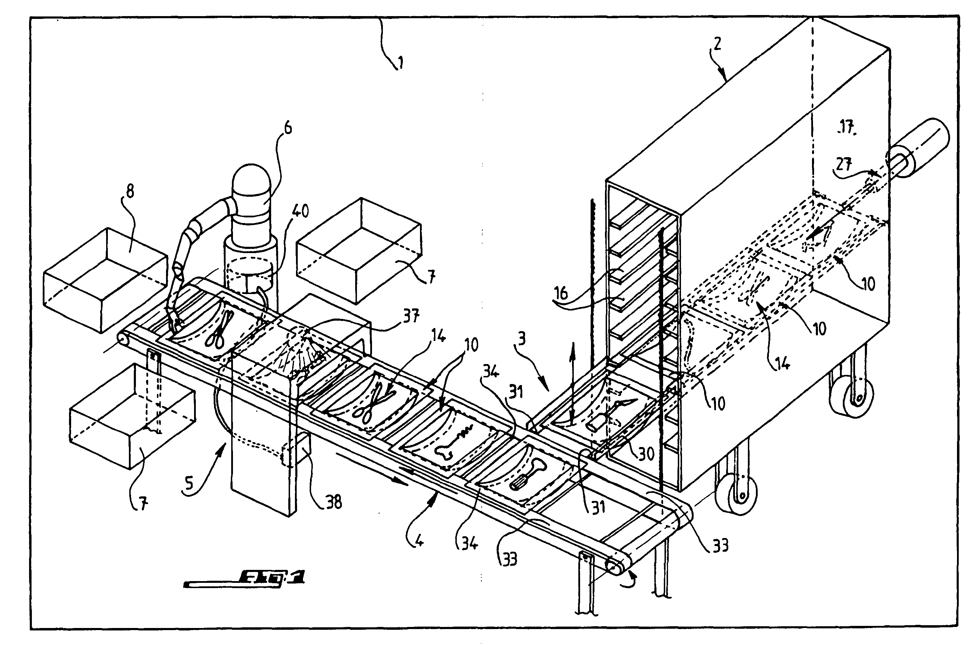

En se reportant à la figure 1, on constate qu'une installation selon l'invention, pour le tri des instruments nécessaires à une opération chirurgicale, comporte essentiellement, disposé à l'intérieur d'une enceinte à ambiance blanche 1, c'est-à-dire d'une propreté la plus parfaite possible, essentiellement un dispositif de stockage de l'ensemble des instruments chirurgicaux susceptibles d'être utilisés pour les différentes opérations chirurgicales possibles, à l'état propre, un dispositif 3 de transfert des instruments à un convoyeur 4 destiné à les transporter à un poste 5 de reconnaissance de la nature ou du type des instruments et un mécanisme de rangement des instruments après leur reconnaissance dans des récipients 7 dont chacun est destiné à contenir les instruments devant être utilisés pour une opération prédéterminée, un récipient 8 étant prévu pour la réception d'instruments jugés non conformes aux exigences établies pour les opérations chirurgicales.

Le dispositif 2 de stockage de l'ensemble des instruments est réalisé, dans le cas d'exemple, sous forme d'un chariot comportant un certain nombre de niveaux 9 de retenue chacun d'une pluralité de plateaux 10, dans l'exemple représenté 3, dont chacun peut comporter une pluralité d'alvéoles 12 de logement d'un instrument chirurgical 14. Dans le cas d'espèce, pour des raisons de simplification des dessins, chaque plateau ne comporte qu'une alvéole. Les plateaux 10 de chaque niveau 9 du chariot de stockage 2 sont supportés par des éléments de support en forme de rail de glissement 16 fixé chacun à une paroi latérale 17 du chariot orienté en direction du convoyeur 4.

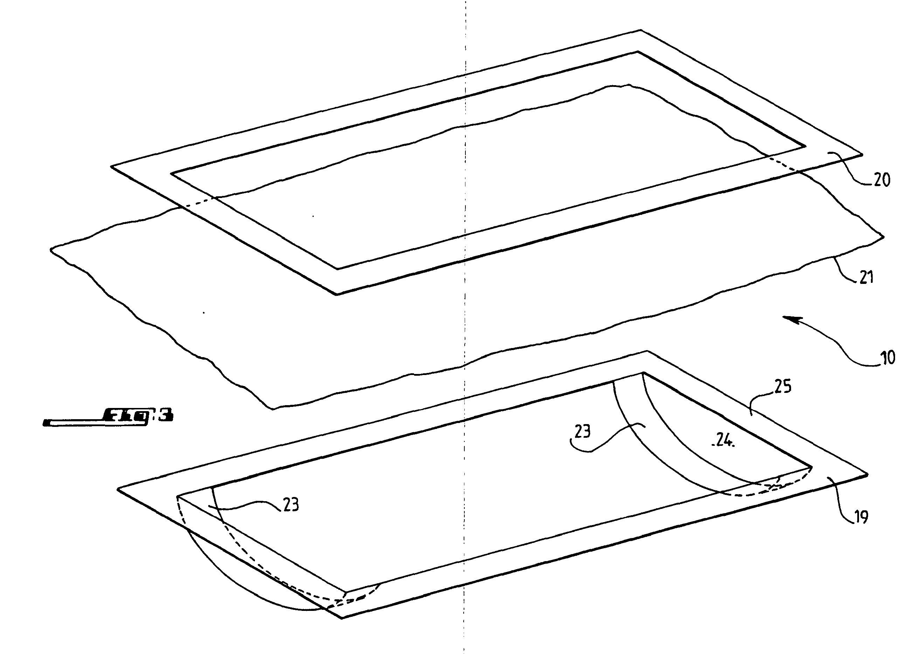

En se reportant à la figure 3, on constate que chaque plateau 10 se compose de deux cadres superposés, en un matériau facilement nettoyable, tel que de l'acier inoxydable, à savoir un élément de cadre inférieur 19 de forme générale rectangulaire et un cadre supérieur 20 de forme complémentaire et susceptible d'être fixé sur le cadre inférieur, par des moyens en forme de clips (non représentés), susceptibles de serrer le cadre supérieur sur le cadre inférieur pour qu'un élément jetable 21, en un matériau souple transparent aux rayons X avantageusement en papier ou en tissu, pourrait être inséré à deux de ses bords opposés entre les bords correspondants des deux cadres, pour former une alvéole 12 de logement d'un instrument 14. Pour la formation aisée des alvéoles et leur maintien, le cadre inférieur 19 est pourvu d'éléments de support 23 en forme d'arcs, au niveau de chaque extrémité longitudinale. Il est avantageux que l'alvéole soit fermée à chaque extrémité longitudinale par une paroi verticale 24 formant l'espace entre le cadre et l'arc correspondant 23.

Comme on le voit sur la figure 1, chaque plateau 10 est déplaçable dans le chariot 2, perpendiculairement à son axe longitudinal en prenant appui par les côtés courts 25 sur les rails de glissement 16 du chariot.

L'agencement de transfert des plateaux 10 de chaque étage du chariot comporte, pour pousser les plateaux hors du chariot, un dispositif poussoir 27 et monté verticalement mobile à l'arrière du chariot 2, pour être positionnable à chaque niveau 9 du chariot. Le dispositif comporte un piston poussoir d'un vérin hydraulique qui, lors de son mouvement de sortie, pousse le dernier plateau et déplace ainsi l'ensemble de plateaux en direction du convoyeur.

Le dispositif de transfert 3 comporte en outre, à l'avant du chariot de stockage 2 de plateaux 10 un cadre 30 de transfert des plateaux 10 du chariot 2 au convoyeur 4, qui est verticalement déplaçable pour pouvoir recevoir les plateaux de chaque niveau 9 de stockage du chariot et pour les transporter ensuite à la hauteur du convoyeur 4 pour que les plateaux puissent être posés sur ce dernier. Plus précisément, dans l'exemple représenté, le cadre de transfert comporte essentiellement deux rails de coulissement 31, chacun étant susceptible d'être aligné, dans une position de réception de plateau d'un niveau ou étage 9, à un rail de glissement 16 de niveau, de façon que les plateaux 10 puissent être déplacés, sous l'effet du dispositif poussoir 27 des rails 16 du chariot aux rails 31 du cadre de transfert 30. Le cadre de transfert est dimensionné de façon à transférer successivement les plateaux 10 au convoyeur 4.

Le convoyeur 4 est montré, sur la figure 1, comme étant réalisé sous forme d'une bande de transport sans fin comportant, essentiellement deux brins plats parallèles 33, destinés au transport des plateaux 10, reliés par des traverses 34.

Pour assurer le transfert des plateaux 10 du cadre de transfert 30 aux brins de convoyeur 33, les rails de support 31 du cadre de transfert s'étendent jusqu'au-dessus du convoyeur et sa portion d'appui de plateau, dans sa position avant de transfert, est abaissable pour permettre la pose des chariots sur les brins du convoyeur situés en dessous. Puis ils s'écartent latéralement et reviennent dans leur position de réception d'un nouveau plateau.

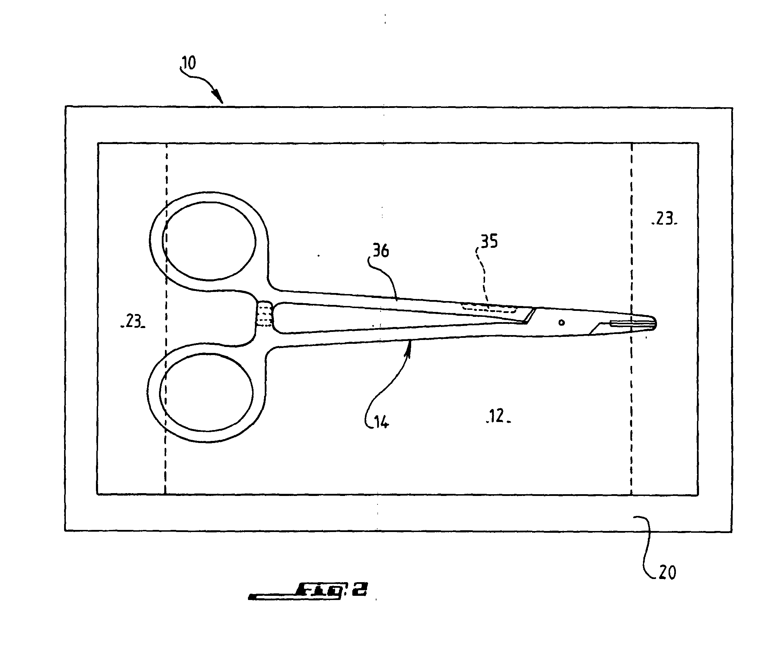

Le convoyeur 4 transporte les plateaux reçus du chariot de stockage 2, par l'intermédiaire du cadre de transfert 3 au poste de reconnaissance 5 des instruments placés dans les alvéoles 12 des plateaux 10. Concernant les instruments chirurgicaux, de fonctions et de formes différentes, ils sont chacun pourvus d'un code d'identité, reconnaissable par le poste 5 de façon que celui-ci soit en mesure de distinguer les instruments apportés par le convoyeur 4 selon leur type spécifique. Plus précisément, le code d'identité de chaque instrument est marqué, sous toute forme appropriée, sur un insert 35 qui est placé dans une cavité appropriée usinée dans l'instrument et fermée ensuite. La figure 2 montre à titre d'exemple, une pince chirurgicale dans une des branches notée 36 de laquelle est incorporé un insert 35.

Le poste de reconnaissance des instruments est avantageusement un lecteur aux rayons X, en forme d'un portique, à travers lequel passe le convoyeur 4 et qui comporte, disposée au-dessus du convoyeur 4, une source de rayons X 37, tandis qu'un récepteur des rayons ayant traversé l'instrument 14 est disposé sous le convoyeur.

Pour pouvoir reconnaître le type d'un instrument, d'après son insert, les éléments de celui-ci, qui constituent le code d'identification, doivent être moins transparents aux rayons X que le matériau constitutif de l'instrument. Ce code pourrait résider dans la forme de l'insert ou des marques prévues sur celui-ci. Les inserts sont avantageusement réalisés en un matériau relativement opaque aux rayons X, tel que le laiton ou un alliage de laiton. Pour assurer une reconnaissance fiable des instruments, il est nécessaire que les instruments soient présentés au poste de reconnaissance dans une position toujours clairement définie.

Après la reconnaissance des instruments, par lecture de leurs inserts, à l'aide des rayons X, l'appareil de rangement 6 saisit les instruments et les range dans les récipients 7 en forme de boîte, sous les ordres d'un dispositif informatique 40.

Ce dispositif comporte, dans sa mémoire, des protocoles d'opération, un protocole pour chaque type d'opération, qui indique les instruments devant être utilisés au cours de l'opération, le cas échéant dans leur ordre d'utilisation. Etant donné qu'à chaque type d'opération correspond un boîtier 7, les instruments devant être rangés dans cette boîte sont indiqués par le protocole établi pour cette opération. A cette fin, le dispositif informatique 40 identifie tout d'abord, d'après le signal qu'il vient de recevoir du lecteur 38 du poste de reconnaissance, le type de l'instrument qui vient d'être examiné et détermine, en se reportant aux différents protocoles à quel type d'opération et ainsi à quelle boîte 7 un instrument de ce type est destiné. Puis, il donne l'ordre au dispositif de rangement 6 de saisir l'instrument identifié dans le plateau et de le ranger dans la boîte appropriée.

En comparant les instruments rangés dans une boîte à ceux figurant dans le protocole, le dispositif informatique connaît à tout moment l'état de "remplissage" de chaque boîte 7. S'il constate qu'une boîte est terminée, c'est-à-dire contient tous les instruments nécessaires pour l'opération considérée, la boîte est fermée, par exemple par la mise en place de son couvercle.

L'invention prévoit également la possibilité d'écarter des instruments jugés non aptes à être utilisés, du circuit d'utilisation, en les plaçant dans une boîte de rebus 8. Diverses raisons pourraient motiver cette mesure, par exemple l'usure d'un instrument, l'impossibilité de l'identifier ou parce qu'il s'agit d'un instrument souillé.

Concernant le poste de rangement 6, des appareils susceptibles de fonctionner de la manière décrite plus haut, sont largement connus, si bien qu'il n'est pas nécessaire de décrire l'appareil utilisé dans le cadre de l'invention, précisément. Il convient d'indiquer qu'un tel appareil comporte un bras robot capable de saisir les instruments dans leur plateau et de les placer ensuite, en fonction des instructions reçues du dispositif informatique, dans les boîtes appropriées.

Concernant le fonctionnement de l'invention et le déroulement du procédé ainsi que les différentes étapes de celui-ci, ils découlent de la description qui vient d'être faite. Il va sans le préciser davantage, que pour chaque transfert d'un plateau sur le convoyeur, celui-ci est arrêté pendant un bref instant de temps nécessaire à la pose du plateau. Les arrêts du convoyeur pour le chargement des plateaux et le processus de reconnaissance des instruments par le poste de reconnaissance ainsi que le rangement des instruments sont coordonnés par le dispositif informatique.

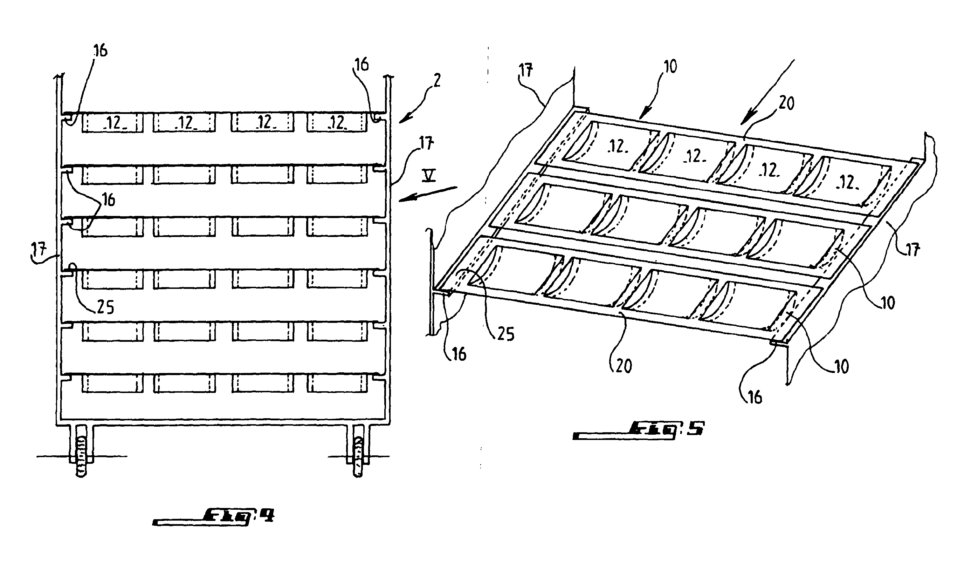

La description de l'invention, qui vient d'être faite, n'a été donnée qu'à titre d'exemple et des multiples modifications peuvent être apportées sans sortir du cadre de l'invention. Pour augmenter la capacité de stockage du chariot 2, chaque plateau 10 pourrait comporter quatre alvéoles, comme le montrent les figures 4 et 5. La flèche symbolise l'action du mécanisme de déplacement du plateau vers le convoyeur 3.

Il est à noter que l'utilisation comme moyen de lecture de rayons X permet de placer les éléments de code à l'intérieur de l'instrument, de façon inaccessible de l'extérieur. Par conséquent les informations de code sont inviolables et inaltérables. La lecture à l'aide de rayons X exclut donc toute erreur de lecture qui pourrait être occasionnée par des phénomènes d'usure, de corrosion ou d'autres modifications de la surface extérieure des instruments. La lecture est également totalement insensible à des dépôts de saleté sur les instruments. D'autre part, lorsqu'il s'agit d'instruments circulaires qui peuvent se présenter dans des positions angulaires différentes, il suffit de faire en sorte que l'insert comporte un profil de rainures circulaires pour que la lecture soit toujours correcte indépendamment de la position en rotation de l'instrument. Par contre lorsqu'il s'agit d'instruments plats, il suffit que le profil de code soit usiné dans l'insert dans le plan de l'instrument pour que la lecture soit toujours correcte.

## Revendications

1. Procédé de tri et de rangement d'instruments tels que des instruments chirurgicaux, devant être utilisés pour une opération prédéterminée, telle qu'une opération chirurgicale, **caractérisé en ce que** l'on établit pour chaque opération parmi une pluralité d'opérations susceptibles d'être effectuées, un protocole d'opération indiquant au moins les instruments (14) devant être utilisés, que l'on associe à chaque instrument (14) d'un ensemble d'instruments susceptibles d'être utilisés au cours de ladite pluralité d'opérations, un insert (35) porteur d'un code d'identification de l'instrument, et accomplit le tri des instruments nécessaires pour une opération prédéterminée, dans une enceinte à ambiance blanche (1) en assurant le transport des instruments (14) d'une zone de stockage à l'état propre, à un poste de reconnaissance (5) par lecture de leur codé et le rangement des instruments après leur identification en fonction des protocoles d'opération dans des récipients (7) dont chacun continent les instruments nécessaires (14) pour une opération, que l'insert (35) est réalisé en un matériau plus opaque aux rayons x que le matériau constitutif des instruments et **en ce que** la reconnaissance des instruments par la lecture du code d'identification des inserts (35) est effectuée à l'aide de rayons X.

2. Procédé selon la revendication 1, **caractérisé en ce que** l'on place l'insert (35) dans une capité (36) pratiquée dans l'instrument (14), que l'on ferme ensuite

3. Procédé selon l'une des revendications 1ou 2, **caractérisé en ce qu'**après la reconnaissance d'un instrument (14) par lecture de son code d'identification, on détermine, en consultant les protocoles d'opération, une opération à laquelle cet instrument pourrait être utilisé.

4. procédé selon la revendication 3, **caractérisé en ce que** l'on fait ranger l'instrument (14) qui a été reconnu dans un récipient (7) destiné à contenir les instruments destinés à l'opération déterminée.

5. Installation pour la mise en oeuvre du procédé selon l'une des revendications 1 à 4, **caractérisée en ce qu'**elle comporte, à l'intérieur d'une enceinte à ambiance blanche (1), un dispositif (2) de stockage d'instruments propres (14), un dispositif (3, 4) de transport des instruments à un poste (5) de reconnaissance par lecture à l'aide des rayons x des instruments pourvus d'un insert plus opaque aux rayons X que le matériau constitutif des instruments (14), un mécanisme (6) de rangement des instruments reconnus (14) dans des récipients (7) dont chacun comporte des instruments destinés à une opération, en fonction des protocoles d'opération, et un dispositif informatique (40) de commande du mécanisme de rangement.

6. Installation selon la revendication 5,
**caractérisée en ce que** le dispositif de stockage des instruments propres (14) est réalisé sous forme d'un meuble de stockage comportant une pluralité de niveaux (9) de stockage, chacun d'au moins un plateau (10) de logement d'un instrument (14).

7. Installation selon la revendication 6,
**caractérisée en ce que** le meuble de stockage comporte un mécanisme (27) d'évacuation des plateaux (10) des différents niveaux de stockage (9), sur un mécanisme (30) de transfert des plateaux évacués sur le convoyeur (4), les mécanismes d'évacuation et de transfert étant déplaçables en hauteur pour desservir les différents niveaux de stockage (9).

8. Installation selon la revendication 7,
**caractérisée en ce que** chaque niveau (9) de stockage de plateaux (10) comporte des rails (16) sur lesquels peuvent glisser les plateaux (10) et **en ce que** le mécanisme de transfert (30) déplaçable en hauteur comporte des rails (31) de glissement de plateau qui sont alignables avec les rails de glissement (16) du meuble de stockage.

9. Installation selon la revendication 8,
**caractérisée en ce que** les rails de glissement (31) du mécanisme de transfert (30) s'étendent jusqu'au-dessus du convoyeur (4) et **en ce que** la partie d'extrémité des rails, située au-dessus du convoyeur, est abaissable et écartable pour permettre la pose du plateau (10) qu'elle supporte sur le convoyeur.

10. Installation selon l'une des revendications 7 à 9, **caractérisée en ce que** le mécanisme (27) d'évacuation des plateaux (10) comporte des moyens poussoirs exerçant une poussée en direction du mécanisme de transfert (3) sur au moins un plateau occupant un étage de stockage

11. Installation selon l'une des revendications 7 à 10, **caractérisée en ce qu'**un étage de stockage (9) contient une pluralité de plateaux (10).

12. Installation selon l'une des revendications 6 à 11, **caractérisée en ce que** le meuble de stockage (2) est réalisé sous forme d'un chariot.

13. Installation selon l'une des revendications 6 à 12, **caractérisée en ce qu'**un plateau (10) comporte un cadre inférieur (19) et un cadre supérieur (20) de formes complémentaires adaptés pour être fixés par serrage sur le cadre inférieur et un élément souple (21), tel qu'une feuille de papier ou un tissu, qui est disposé entre les deux cadres (19) et (20) de façon à former une alvéole (12) de logement d'un instrument (14).

14. Installation selon la revendication 18,
**caractérisée en ce que** les cadres (19, 20) sont réalisés en un matériau nettoyable tel que de l'acier inox et l'élément (21) de formation de l'alvéole est un matériau jetable.

15. Installation selon l'une des revendications 5 à 14, **caractérisée en ce que** le poste (5) de reconnaissance des instruments (14) comporte une source de rayons X (37) disposée au-dessus du convoyeur (4) et un récepteur (38) des rayons ayant traversé un plateau (10) de logement d'un instrument (14), dans sa position de reconnaissance à l'intérieur du poste de reconnaissance (5), disposé sous le convoyeur.

16. Installation selon l'une des revendications 5 à 15, **caractérisée en ce qu'**elle est adaptée pour reconnaître des instruments (14) devant être écartés et **en ce qu'**il comporte un récipient (8) de réception des instruments écartés.

17. Installation selon l'une des revendications 5 à 16, **caractérisée en ce que** le dispositif informatique est adapté pour coordonner le fonctionnement des différents dispositifs et mécanismes de l'installation.

18. Installation selon l'une des revendications 5 à 17, **caractérisée en ce qu'**un instrument (14) comporte un insert (35) disposé dans une cavité (36) fermée de l'instrument et portant des éléments de codage indicateurs de la nature de l'instrument, l'insert étant réalisé en un matériau plus opaque aux rayons x que le matériau constitutif de l'instrument.

## Claims

1. A method for sorting and arranging instruments, such as surgical instruments, to be used for a predetermined operation, such as a surgical operation, **characterized in that** for each operation from a plurality of operations likely to be performed, an operating protocol is set up specifying at least the instruments (14) to be used, **in that** with each instrument (14) of a set of instruments likely to be used during said plurality of operations an insert (35) bearing a code identifying the instrument is associated, and **in that** sorting of the instruments required for a predetermined operation is done in a white room (1), with transport of the instruments (14) in a clean state from a storage area to a station (5) for the code thereof to be recognized by reading, and arranging the instruments after they have been identified depending on the operating protocols in containers (7), each of which contains the instruments (14) required for one operation being ensured, **in that** the insert (35) is made from a material which is more radiopaque than the material composing the instruments, and **in that** recognition of the instruments by reading the identification code of the inserts (35) is done by means of X-rays.

2. The method according to claim 1, **characterized in that** the insert (35) is placed into a cavity (36) made in the instrument (14), and it is closed thereafter.

3. The method according to any of claims 1 or 2, **characterized in that** after recognition of an instrument (14) by reading the identification code thereof, an operation for which the instrument could be used is determined by consulting the operating protocols.

4. The method according to claim 3, **characterized in that** the instrument (14) having been recognized is arranged within a container (7) designed for holding the instruments for the determined operation.

5. An installation for implementing the method according to any of claims 1 to 4, **characterized in that** it comprises, within a white room (1), a device (2) for storing clean instruments (14), a device (3, 4) for transporting instruments to a station (5) for recognizing through reading using X-rays instruments provided with an insert more radiopaque than the material composing the instruments (14), a mechanism (6) for arranging the instruments (14) recognized within containers (7), each of which comprises instruments for one operation, depending on the operating protocols, and a computer device (40) for controlling the arranging mechanism.

6. The installation according to claim 5, **characterized in that** the device for storing clean instruments (14) is embodied as a storage cabinet comprising a plurality of storage levels (9), each composed of at least one tray (10) for receiving one instrument (14).

7. The installation according to claim 6, **characterized in that** the storage cabinet comprises a mechanism (27) for discharging the trays (10) from the various storage levels (9), onto a mechanism (30) for transferring discharged trays onto the conveyor (4), the discharge and transport mechanisms being mobile in the height thereof in order to serve the various storage levels (9).

8. The installation according to claim 7, **characterized in that** each level (9) storing trays (10) comprises rails (16) on which the trays (10) may slide, and **in that** the transfer mechanism (30) mobile in the height thereof comprises tray slide rails (31), which can be aligned on the slide rails (16) of the storage cabinet.

9. The installation according to claim 8, **characterized in that** the slide rails (31) of the transfer mechanism (30) extend above the conveyor (4), and **in that** the end part of the rails, located above the conveyor, can be lowered and spread apart to allow for the tray (10) supported thereby to be placed on the conveyor.

10. The installation according to any of claims 7 to 9, **characterized in that** the mechanism (27) for discharging the trays (10) comprises pushing means pushing in the direction of the transfer mechanism (3) on at least one tray occupying one storage stage.

11. The installation according to any of claims 7 to 10, **characterized in that** one storage stage (9) contains a plurality of trays (10).

12. The installation according to any of claims 6 to 11, **characterized in that** the storage cabinet (2) is embodied as a carriage.

13. The installation according to any of claims 6 to 12, **characterized in that** one tray (10) comprises a lower frame (19) and an upper frame (20) having complementary shapes adapted to be fastened by tightening on the lower frame, and a flexible element (21), such as a sheet of paper or fabric, which is arranged between both frames (19) and (20) so as to form a compartment (12) for housing an instrument (14).

14. The installation according to claim 12,
**characterized in that** the frames (19, 20) are made of cleanable material, such as stainless steel, and the element (21) for forming the compartment is a disposable material.

15. The installation according to any of claims 5 to 14, **characterized in that** the station (5) for recognizing the instruments (14) comprises a source of X-rays (37) arranged above the conveyor (4), and a receiver (38) of the rays having gone through a tray (10) housing an instrument (14), in the recognition position thereof within the recognition station (5), and which is arranged under the conveyor.

16. The installation according to any of claims 5 to 15, **characterized in that** it is adapted for recognizing instruments (14) to be rejected, and **in that** it comprises a container (8) for receiving rejected instruments.

17. The installation according to any of claims 5 to 16, **characterized in that** the computer device is adapted for coordinating the operation of the various devices and mechanisms of the installation.

18. The installation according to any of claims 5 to 17, **characterized in that** one instrument (14) comprises an insert (35) arranged in a closed cavity (36) of the instrument, and bearing code elements indicating the nature of the instrument, with the insert being made of a material, which is more radiopaque than the material composing the instrument.

## Patentansprüche

1. Verfahren zum Sortieren und Aufbewahren von Instrumenten, wie etwa chirurgischen Instrumenten, die für eine vorherbestimmte Operation, wie etwa eine chirurgische Operation, zu verwenden sind, **dadurch gekennzeichnet, dass** für jede Operation aus einer Vielzahl von Operationen, die ausgeführt werden können, ein Operationsprotokoll aufgestellt wird, das mindestens die zu verwendenden Instrumente (14) angibt, dass jedem Instrument (14) eines Satz von Instrumenten, die während der Vielzahl von Operationen verwendet werden können, ein Einsatzteil (35) zugeordnet wird, das einen Code trägt, der das Instrument identifiziert, und dass das Sortieren der für eine vorherbestimmte Operation benötigten Instrumente in einem Reinraum (1) erfolgt, wobei der Transport der Instrumente (14) von einem Lagerbereich in sauberem Zustand bis zu einer Station (5) zum Erkennen ihres Codes durch Ablesen und das Aufbewahren der Instrumente nach ihrer Identifizierung in Abhängigkeit von den Operationsprotokollen in Behältern (7), von denen jeder die Instrumente (14) enthält, die für eine Operation benötigt werden, sichergestellt wird, dass das Einsatzteil (35) aus einem Material hergestellt wird, das strahlungsundurchlässiger ist als das Material, aus dem die Instrumente bestehen, und dass das Erkennen der Instrumente durch Ablesen des Identifizierungscodes des Einsatzteils (35) anhand von Röntgenstrahlen erfolgt.

2. Verfahren nach Anspruch 1, **dadurch gekennzeichnet, dass** das Einsatzteil (35) in einen Hohlraum (36) gesetzt wird, der in dem Instrument (14) eingebracht ist, und dass es anschließend geschlossen wird.

3. Verfahren nach einem der Ansprüche 1 oder 2, **dadurch gekennzeichnet, dass** nach dem Erkennen eines Instruments (14) durch Ablesen seines Identifizierungscodes, unter Abfragen der Operationsprotokolle eine Operation bestimmt wird, für die dieses Instrument verwendet werden könnte.

4. Verfahren nach Anspruch 3, **dadurch gekennzeichnet, dass** das Instrument (14), das erkannt wurde, in einem Behälter (7) aufbewahrt wird, der dazu gedacht ist, die Instrumente zu enthalten, die für die bestimmte Operation gedacht sind.

5. Anlage zum Durchführen des Verfahrens nach einem der Ansprüche 1 bis 4, **dadurch gekennzeichnet, dass** sie innerhalb eines Reinraums (1) eine Vorrichtung (2) zum Lagern von sauberen Instrumenten (14), eine Vorrichtung (3, 4) zum Transportieren von Instrumenten bis zu einer Station (5) zum Erkennen durch Ablesen anhand von Röntgenstrahlen der Instrumente, die mit einem Einsatzteil versehen sind, das strahlungsundurchlässiger ist als das Material, aus dem die Instrumente (14) bestehen, einen Mechanismus (6) zum Aufbewahren der erkannten Instrumente (14) in den Behältern (7), von denen jeder Instrumente umfasst, die für eine Operation gedacht sind, in Abhängigkeit von den Operationsprotokollen, und eine Computervorrichtung (40) zum Steuern des Lagermechanismus umfasst.

6. Anlage nach Anspruch 5, **dadurch gekennzeichnet, dass** die Vorrichtung zum Lagern der sauberen Instrumente (14) als ein Lagerschrank ausgebildet ist, der eine Vielzahl von Lagerebenen (9) umfasst, die jeweils aus mindestens einer Schale (10) zum Aufnehmen eines Instruments (14) bestehen.

7. Anlage nach Anspruch 6, **dadurch gekennzeichnet, dass** der Lagerschrank einen Mechanismus (27) umfasst zum Austragen der Schalen (10) aus den verschiedenen Lagerebenen (9) auf einen Mechanismus (30) zum Übertragen der ausgetragenen Schalen auf eine Fördereinrichtung (4), wobei die Mechanismen zum Austragen und Übertragen in ihrer Höhe bewegbar sind, um die verschiedenen Lagerebenen (9) zu bedienen.

8. Anlage nach Anspruch 7, **dadurch gekennzeichnet, dass** jede Ebene (9) zum Lagern der Schalen (10) Schienen (16) umfasst, auf denen die Schalen (10) gleiten können, und dass der Übertragungsmechanismus (30), der in seiner Höhe bewegbar ist, Schalengleitschienen (31) umfasst, die mit den Gleitschienen (16) des Lagerschranks ausgerichtet werden können.

9. Anlage nach Anspruch 8, **dadurch gekennzeichnet, dass** die Gleitschienen (31) des Übertragungsmechanismus (30) sich oberhalb der Fördereinrichtung (4) erstrecken, und dass der Endteil der Schienen, der sich oberhalb der Fördereinrichtung befindet, abgesenkt und gespreizt werden kann, um das Aufsetzen der davon getragenen Schale (10) auf die Fördereinrichtung zu ermöglichen.

10. Anlage nach einem der Ansprüche 7 bis 9, **dadurch gekennzeichnet, dass** der Mechanismus (27) zum Austragen der Schalen (10) Stoßmittel umfasst, die einen Schub in Richtung auf den Übertragungsmechanismus (3) auf mindestens eine Schale ausüben, die eine Lageretage einnimmt.

11. Anlage nach einem der Ansprüche 7 bis 10, **dadurch gekennzeichnet, dass** eine Lageretage (9) eine Vielzahl von Schalen (10) enthält.

12. Anlage nach einem der Ansprüche 6 bis 11, **dadurch gekennzeichnet, dass** der Lagerschrank (2) als Schlitten ausgebildet ist.

13. Anlage nach einem der Ansprüche 6 bis 12, **dadurch gekennzeichnet, dass** eine Schale (10) einen unteren Rahmen (19) und einen oberen Rahmen (20), die ergänzend geformt sind und dazu geeignet sind, durch Klemmen auf dem unteren Rahmen befestigt zu werden, und ein biegsames Element (21), wie etwa ein Bogen Papier oder Stoff, der zwischen den beiden Rahmen (19) und (20) angeordnet wird, um ein Fach (12) zum Aufnehmen eines Instruments (14) zu bilden, umfasst.

14. Anlage nach Anspruch 12, **dadurch gekennzeichnet, dass** die Rahmen (19, 20) aus einem Material ausgebildet sind, das gereinigt werden kann, wie etwa rostfreier Stahl, und das Element (21) zum Bilden des Fachs ein Wegwerfmaterial ist.

15. Anlage nach einem der Ansprüche 5 bis 14, **dadurch gekennzeichnet, dass** die Station (5) zum Erkennen der Instrumente (14) eine Quelle von Röntgenstrahlen (37), die oberhalb der Fördereinrichtung (4) angeordnet ist, und einen Empfänger (38) für die Strahlen, die durch eine Schale (10) zum Aufnehmen eines Instruments (14) in seiner Erkennungsposition im Innern der Erkennungsstation (5) gegangen sind, und der unter der Fördereinrichtung angeordnet ist, umfasst.

16. Anlage nach einem der Ansprüche 5 bis 15, **dadurch gekennzeichnet, dass** sie dazu geeignet ist, um Instrumente (14) zu erkennen, die auszuschließen sind, und dass sie einen Behälter (8) zum Aufnehmen der ausgeschlossenen Instrumente umfasst.

17. Anlage nach einem der Ansprüche 5 bis 16, **dadurch gekennzeichnet, dass** die Computervorrichtung dazu geeignet ist, um den Betrieb der verschiedenen Vorrichtungen und Mechanismen der Anlage zu koordinieren.

18. Anlage nach einem der Ansprüche 5 bis 17, **dadurch gekennzeichnet, dass** ein Instrument (14) ein Einsatzteil (35) umfasst, das in einem geschlossenen Hohlraum (36) des Instruments angeordnet ist und Codierelemente trägt, welche die Beschaffenheit des Instruments angeben, wobei das Einsatzteil aus einem Material ausgebildet ist, das strahlungsundurchlässiger ist als das Material, aus dem das Instrument besteht.
